# EUROPEAN PATENT APPLICATION

(11) **EP 4 368 179 A1**
(43) Date of publication of application: **15.05.2024**
(21) Application number: 22837063.1
(22) Date of filing: 08.07.2022
(51) Int. Cl.: A61K 31/192, A61K 9/08, A61K 9/06, A61K 9/70, A61K 47/36, A61K 47/26, A61P 27/02

(54) **COMPOSITION CONTAINING LOXOPROFEN SODIUM**

(30) Priority: 09.07.2021 CN 202110780094
(71) Applicant: Guangzhou Ocusun Ophthalmic Biotechnology Co., Ltd., Guangzhou, Guangdong 511400 (CN); Ocusun Ophthalmic Pharmaceutical (Guangzhou) Co., Ltd., Guangzhou, Guangdong 511400 (CN)
(72) Inventor: ZHOU, Shengan, Guangzhou, Guangdong 511400 (CN); YU, Chuiliang, Guangzhou, Guangdong 511400 (CN); WANG, Yandong, Guangzhou, Guangdong 511400 (CN); FENG, Ximing, Guangzhou, Guangdong 511400 (CN); LI, Yaxiong, Guangzhou, Guangdong 511400 (CN); WU, Meirong, Guangzhou, Guangdong 511400 (CN)
(74) Representative: Murgitroyd & Company
(86) International application number: PCT/CN2022/104726
(87) International publication number: WO 2023/280318

(57) **Abstract**

A composition containing loxoprofen sodium. The composition comprises loxoprofen sodium, sodium hyaluronate and polysorbate 80, wherein the molecular weight of sodium hyaluronate is 0.8 × 10⁶ - 1.6 × 10⁶. The composition has good stability and a long shelf life and is convenient to use.

## Description

The present application claims the priority of Chinese Patent Application No. 202110780094.9 filed on July 09, 2021. The contents of the Chinese patent application are incorporated herein by reference in their entireties.

### TECHNICAL FIELD

The present invention relates to the field of medicine, and particularly to a composition containing loxoprofen sodium.

### BACKGROUND

Non-steroidal anti-inflammatory drugs (NSAIDS) have anti-inflammatory, antipyretic and analgesic effects, without the shortcoming of glucocorticoids drugs that are easy to cause more serious adverse reactions, and have become a class of important anti-inflammatory drugs in ophthalmological clinics. Eye drops of the non-steroidal anti-inflammatory drugs are used for treating eye traumas, have the functions of diminishing inflammations after laser surgery and cataract surgery, and inhibiting pupil shrinkage during surgery, and achieve good effects.

Loxoprofen sodium (chemical name: sodium 2-[4-(2-oxocyclopentyl-1-methyl)phenyl]propionate dihydrate) is a non-steroidal anti-inflammatory drug, which has an action mechanism of inhibiting prostaglandin synthesis and targets cyclooxygenase. At present, this compound is clinically used for resisting inflammations and relieving pain for rheumatoid arthritis, osphyalgia, scapulohumeral periarthritis, neck, shoulder and wrist syndromes and the like, relieving pain and diminishing inflammations after surgery, trauma and tooth extraction, clearing away heat and relieving pain for acute upper respiratory tract inflammations, and the like.

In practical applications, eye drops containing loxoprofen sodium may have poor chemical stability, especially high content of ring-opening impurities, in the case of long-term storage.

### CONTENT OF THE PRESENT INVENTION

An objective of the present invention is to overcome the defects of the prior art and provide a composition containing loxoprofen sodium with good stability and long shelf life.

Specifically, the present invention provides a composition containing loxoprofen sodium. The composition includes loxoprofen sodium, sodium hyaluronate, and polysorbate 80, wherein the sodium hyaluronate has a molecular weight of 0.8×10⁶-1.6×10⁶.

The present invention has creatively found that the combination of sodium hyaluronate of specific molecular weight and polysorbate 80 with loxoprofen sodium can significantly improve the chemical stability of the loxoprofen sodium itself, so that a composition with more stable quality can be obtained.

The sodium hyaluronate used in the present invention is in a sodium salt form of hyaluronic acid (HA). Hyaluronic acid is a homopolymer formed by repeated alternation of D-glucuronic acid and N-acetylglucosamine disaccharide units, and has a weight-average molecular weight being generally 100,000 to 10,000,000 Da. At present, the average molecular weight of HA used in clinical sodium hyaluronate preparations, such as viscoelastic agents for ophthalmic surgery, intra-articular injections for the treatment of arthritis, postoperative anti-adhesion preparations and eye drops, is generally 500,000 to 5,000,000 Da. The present invention has found that the sodium hyaluronate having a molecular weight in the range of 0.8×10⁶-1.6×10⁶ is used in combination with polysorbate 80, which can significantly improve the stability of loxoprofen sodium.

In some embodiments, the sodium hyaluronate has a molecular weight of 0.8×10⁶-1.4×10⁶. Specifically, in the composition provided by the present invention, the sodium hyaluronate may have a molecular weight of 0.8×10⁶-1.0×10⁶, or 1.0×10⁶-1.2×10⁶, or 1.2×10⁶-1.4×10⁶. The molecular weight of the sodium hyaluronate in the present invention refers to a weight-average molecular weight (M_{w}), with a unit of Dalton (Da).

In some embodiments, the composition has a mass ratio of the loxoprofen sodium to the sodium hyaluronate to the polysorbate 80 of (1-4): (0.5-2): (0.1-1), preferably, the ratio is 1: (0.5-1.5): (0.1-1), and more preferably, the ratio is 1: (0.8-1.2): (0.2-0.5).

In some embodiments, the composition further contains one or more of a metal ion complexing agent, an osmotic pressure regulator, a pH regulator, a surfactant, and a bacteriostatic agent.

In some embodiments, the composition containing the loxoprofen sodium also contains, at a minimum, a metal ion complexing agent. The composition preferably has a mass ratio of the loxoprofen sodium to the metal ion complexing agent of (1-4): (0.05-0.2), and more preferably, the ratio is 1: (0.05-0.1).

In some embodiments, the metal ion complexing agent is disodium edetate.

In some embodiments, the composition containing the loxoprofen sodium also contains, at a minimum, an osmotic pressure regulator. The dosage of the osmotic pressure regulator in the present invention is determined according to actual demands, and the osmotic pressure value of the finished product can be adjusted to a level suitable for use in humans.

In some embodiments, the osmotic pressure regulator is selected from one or more of sodium chloride, mannitol, glucose, and potassium chloride, preferably sodium chloride.

In some embodiments, the composition containing the loxoprofen sodium also contains, at a minimum, a pH regulator. The dosage of the pH regulator in the present invention is determined according to actual demands, and the pH value of the finished product can be adjusted to a level suitable for use in humans.

In some embodiments, the pH regulator is selected from one or more of sodium hydroxide, hydrochloric acid, sodium citrate, citric acid, boric acid, and borax.

In some embodiments, the composition containing the loxoprofen sodium also contains, at a minimum, a bacteriostatic agent. The composition preferably has a mass ratio of the loxoprofen sodium to the bacteriostatic agent of (1-4): (0.05-0.2), and more preferably, the ratio is 1: (0.05-0.1).

In some embodiments, the bacteriostatic agent is selected from one or more of thiomersal, quaternary ammonium salts, Domiphen, chlorhexidine, chlorobutanol, nipagin, and sorbic acid, preferably benzalkonium chloride.

In some embodiments, the composition includes loxoprofen sodium, sodium hyaluronate, disodium edetate, sodium chloride, and polysorbate 80.

In some embodiments, the composition includes the following components in parts by weight:

| | |
|---|---|
| loxoprofen sodium | 1-4; |
| sodium hyaluronate | 0.5-2; |
| disodium edetate | 0.05-0.2; |
| sodium chloride | 4-10; and |
| polysorbate 80 | 0.1-1. |

In some embodiments, the composition includes the following components in parts by weight:

| | |
|---|---|
| loxoprofen sodium | 1; |
| sodium hyaluronate | 0.8-1.2; |
| disodium edetate | 0.05-0.1; |
| sodium chloride | 4-9; and |
| polysorbate 80 | 0.2-0.5. |

In some embodiments, the composition includes loxoprofen sodium, sodium hyaluronate, disodium edetate, sodium chloride, polysorbate 80, and benzalkonium chloride.

In some embodiments, the composition includes the following components in parts by weight:

| | |
|---|---|
| loxoprofen sodium | 1-4; |
| sodium hyaluronate | 0.5-2; |
| disodium edetate | 0.05-0.2; |
| sodium chloride | 4-10; |
| polysorbate 80 | 0.1-1; and |
| benzalkonium chloride | 0.05-0.2. |

In some embodiments, the composition includes the following components in parts by weight:

| | |
|---|---|
| loxoprofen sodium | 1; |
| sodium hyaluronate | 0.8-1.2; |
| disodium edetate | 0.05-0.1; |
| sodium chloride | 4-9; |
| polysorbate 80 | 0.2-0.5; and |
| benzalkonium chloride | 0.05-0.1. |

In some embodiments, the composition containing the loxoprofen sodium is in the form of an aqueous solution. The loxoprofen sodium preferably has a concentration of 0.05-5 mg/mL in the aqueous solution, and more preferably, the concentration is 1-2 mg/mL.

In some embodiments, the composition containing the loxoprofen sodium is a solution, such as eye drops.

In some embodiments, the composition containing the loxoprofen sodium is a gelling agent, such as an ophthalmic gel.

In some embodiments, the composition containing the loxoprofen sodium is an ointment, such as an eye ointment.

In a second aspect, the present invention provides a method for preparing the composition.

Specifically, the method for preparing the composition includes the following steps: preparing an aqueous solution of sodium hyaluronate; dissolving other components including loxoprofen sodium in water to obtain a mixture; then mixing the mixture with the aqueous solution of sodium hyaluronate; and adding water to a constant volume.

In the preparation process provided by the present invention, it is advisable to keep a stirring state throughout the whole process to ensure that the sodium hyaluronate is completely dissolved until the solution is clear.

In some embodiments, the composition contains loxoprofen sodium, sodium hyaluronate, polysorbate 80, a metal ion chelating agent, and an osmotic pressure regulator. The method for preparing the composition includes the following steps:
(1) dissolving the sodium hyaluronate with water to obtain a solution I, a temperature of the water used to dissolve the sodium hyaluronate being preferably 40°C or less;
(2) dissolving the loxoprofen sodium with water, and then adding the metal ion chelating agent and the osmotic pressure regulator to obtain a solution II;
(3) adding the solution II to the solution I to obtain a solution III;
(4) dissolving the polysorbate 80 with water to obtain a solution, and adding the obtained solution to the solution III; and
(5) adding water to a constant volume till a full volume.

In some embodiments, the composition contains loxoprofen sodium, sodium hyaluronate, polysorbate 80, a metal ion chelating agent, an osmotic pressure regulator, and a bacteriostatic agent. The method for preparing the composition includes the following steps:
(1) dissolving the sodium hyaluronate with water to obtain a solution I, a temperature of the water used to dissolve the sodium hyaluronate being preferably 40°C or less;
(2) dissolving the loxoprofen sodium with water, and then adding the metal ion chelating agent and the osmotic pressure regulator to obtain a solution II;
(3) adding the solution II to the solution I to obtain a solution III;
(4) dissolving the polysorbate 80 and the bacteriostatic agent respectively with water to obtain two solutions, and adding the obtained two solutions to the solution III; and
(5) adding water to a constant volume till a full volume.

In a third aspect, the present invention provides a use of the composition described in the first aspect in the preparation of a drug for preventing or treating ophthalmic diseases.

In some embodiments, the ophthalmic disease is an inflammation, such as allergic conjunctivitis or uveitis.

In some embodiments, the ophthalmic disease is a dry eye disease, such as a dry eye disease caused by lacrimal gland dysfunction, a dry eye disease caused by reduced tear secretion, a dry eye disease caused by poor tear film stability, or a dry eye disease caused by corneal epithelial cell injury.

In some embodiments, the ophthalmic disease is lacrimal gland dysfunction or is manifested by reduced tear secretion.

In some embodiments, the ophthalmic disease is manifested by poor tear film stability.

In some embodiments, the ophthalmic disease is local edema caused by corneal epithelial cell injury or eye injury.

### DETAILED DESCRIPTION OF THE PREFERRED EMBODIMENT

The following examples are used to illustrate the present invention, but not to limit the scope of the present invention.

In the examples, the mass of loxoprofen sodium is calculated in C₁₅H₁₇NaO₃.

### Example 1

The present embodiment provides an aqueous solution composition containing loxoprofen sodium. The composition consists of the following components: 5 g of loxoprofen sodium, 5 g of sodium hyaluronate having a molecular weight of 0.972×10⁶, 0.5 g of disodium edetate, 42.5 g of sodium chloride, 2.5 g of polysorbate 80, 0.5 g of benzalkonium chloride, and water for injection added to a total of 5 L.

The present embodiment also provides a method for preparing the composition, to be specific:
(1) weighing sodium hyaluronate, taking about 60% of the prescribed amount of water for injection (40 °C or less), and adding sodium hyaluronate while stirring to obtain a solution I;
(2) weighing loxoprofen sodium, adding a small amount of water to dissolve the loxoprofen sodium, adding sodium chloride and disodium edetate and stirring until dissolved to obtain a solution II, adding the solution II to the solution I to obtain a solution III, and stirring continuously;
(3) weighing polysorbate 80, adding a small amount of water for injection, stirring and dissolving to obtain a mixed solution, and then adding the mixed solution to the solution III;
(4) dissolving benzalkonium chloride with 10% of the prescribed amount of water for injection to obtain a solution, adding the solution to the solution III, and continuing to stir until the solution is clear; and
(5) adding water for injection to a constant volume till a full volume.

The above aqueous solution composition may be directly processed into eye drops, which may be filtered and sterilized by a 0.22 µm microporous filter membrane on the basis of adding water for injection to the full amount, and then subpackaged into eye drops bottles in a sterile environment.

### Example 2

The present example provides an aqueous solution composition containing loxoprofen sodium, which differs from Example 1 just in that: the molecular weight of sodium hyaluronate is 1.353×10⁶.

### Example 3

The present example provides an aqueous solution composition containing loxoprofen sodium, which differs from Example 1 just in that: a dosage of loxoprofen sodium is 10 g, and a molecular weight of sodium hyaluronate is 0.878×10⁶.

### Example 4

The present example provides an aqueous solution composition containing loxoprofen sodium, which differs from Example 3 just in that: the molecular weight of sodium hyaluronate is 1.276×10⁶.

### Example 5: Effects on dry eye disease of New Zealand rabbits caused by alkali burns

### 1. Modeling method

Each New Zealand rabbit was anesthetized intravenously with 1% propofol injection (1.5 ml/kg), and 100 µl of 2% lidocaine hydrochloride injection was added dropwise in eyes to anesthetize the surfaces of the eyes after the animal was maintained stable in anesthesia. When the animal was maintained in the third phase of anesthesia, a filter paper strip of about 10 mm×5 mm in size was dipped in 1 mol/L NaOH solution and then placed on the palpebral conjunctiva about 2 mm above the corneal limbus of the rabbit, and the conjunctival sac was repeatedly flushed with about 50 ml of 0.9% sodium chloride injection immediately after 90 s.

### 2. Conjunctival fluorescein staining

2 µl of 0.5% sodium fluorescein solution was added dropwise into the conjunctival sac of the right eye of the New Zealand rabbit by using a pipette, excess fluorescein was rinsed with 0.9% sodium chloride injection after 5 s, and conjunctival staining conditions were observed under a slit lamp microscope with cobalt blue diffuse light. Conjunctival fluorescence staining scoring criteria: The conjunctiva was divided into four quadrants, each of which was graded into four grades according to the degrees and areas of staining: 0 score for no staining, 1 score for scattered punctate staining, 2 scores for dense punctate staining, and 3 scores for sheet staining, for a total of 12 scores (the left eye was not modeled and served as a negative control eye).

### 3. Conjunctival rose bengal staining

2 µl of 1% rose bengal was added dropwise into the conjunctival sac of the right eye of the New Zealand rabbit by using a pipette, excess rose bengal was rinsed with 0.9% sodium chloride injection after 5 s, and conjunctival staining conditions were observed under a slit lamp microscope with green light. The scoring criteria for conjunctival rose bengal staining were consistent with those for conjunctival fluorescence staining criteria.

### 4. Tear secretion amount (both eyes)

The Schirmer I test was used to measure the secretion of tears in New Zealand rabbits. That is, ophthalmic forceps were used to clamp a phenol red thread, place the phenol red thread in the outer canthus of the New Zealand rabbit, and then take the phenol red thread out after 60 seconds, and the length of a wet part of the phenol red thread was measured.

### 5. Tear film break-up time (both eyes)

An adjustable pipette was used to drop 2 µl of 0.5% sodium fluorescein solution into the lower palpebral conjunctival sac of each eye of the New Zealand rabbit, the eyes are blinked manually several times with a constant force, and then the rabbit's eyes were opened with a constant force; the cornea was observed under a slit lamp microscope with cobalt blue light; and when a black area appeared on a green film of the cornea, it was indicated a tear film was broken up. The measurement was performed three times successively, and an average value was taken.

The composition provided in Example 1 of the present invention had a significant improvement effect on a model of rabbit dry eye disease caused by alkali burns, and its improvement effects were mainly manifested in enhancing the tear film stability, increasing the tear secretion amount and alleviating the corneal epithelial cell injury (mainly manifested in reducing the local edema of eye injury).

### Example 6: Irritation test

1. Source of eye drops:
   Pranoprofen Eye Drops: commercially available, the manufacturer is Senju Pharmaceutical Co., Ltd. Fukusaki Plant;
   Diclofenac Sodium Eye Drops: commercially available, the manufacturer is Zhengzhou Zhuofeng Pharmaceutical Co., Ltd.; and
   Loxoprofen Sodium Eye Drops: Example 1 (prepared into eye drops).
2. Experimental operation: 20 patients with dry eye disease in each group were randomly double-blinded with three kinds of eye drops, and the irritation symptoms of Pranoprofen, Diclofenac Sodium and Loxoprofen Sodium Eye Drops each having a concentration of 0.1% for the patients' eyes were compared. Eye irritation symptoms include symptoms such as burning, tingling, or tearing.

According to the eye drops provided in Example 1 of the present invention, some patients had eye irritation symptoms, i.e., tearing symptom with relatively small irritation, but other patients generally reflected strong comfort of the eyes, so this eye drops can be used for a long time, and also more suitable for being applied to children with dry eye disease.

### Example 7: Stability study

The compositions prepared in Example 1 and Example 4 were respectively subject to stability study in an accelerated test for 6 months (40±2 °C/RH 25%±5%) and a long-term test for 24 months (25±2 °C/RH 40%±5%).

In each of the following tables, Impurity 1 refers to:

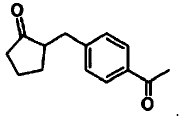

In each of the following tables, a ring-opening impurity refers to:

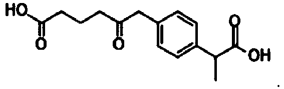

**Table 1: Detection results of related substances in accelerated stability test of the composition of Example 1**

| | Limit requirements | Month 0 | Month 1 | Month 2 | Month 3 | Month 6 |
|---|---|---|---|---|---|---|
| Impurity 1 | ≤1.0% | Not detected | Not detected | Not detected | Not detected | Not detected |
| Ring-opening impurity | ≤1.0% | 0.0128% | 0.0137% | 0.0125% | 0.0162% | 0.0157% |
| Total impurities | ≤2.0% | 0.0579% | 0.0546% | 0.0536% | 0.0465% | 0.0640% |

**Table 2: Detection results of related substances in accelerated stability test of the composition of Example 4**

| | Limit requirements | Month 0 | Month 1 | Month 2 | Month 3 | Month 6 |
|---|---|---|---|---|---|---|
| Impurity 1 | ≤1.0% | Not detected | Not detected | Not detected | Not detected | Not detected |
| Ring-opening impurity | ≤1.0% | 0.0143% | 0.0137% | 0.0128% | 0.0171% | 0.0174% |
| Total impurities | ≤2.0% | 0.0520% | 0.0555% | 0.0489% | 0.0476% | 0.0586% |

**Table 3: Detection results of related substances in long-term stability test of the composition of Example 1**

| | Limit requirements | Month 0 | Month 3 | Month 6 | Month 9 | Month 12 | Month 18 | Month 24 |
|---|---|---|---|---|---|---|---|---|
| Impurity 1 | ≤1.0% | Not detected | Not detected | Not detected | Not detected | Not detected | Not detected | Not detected |
| Ring-opening impurity | ≤1.0% | 0.0128% | 0.0193% | 0.0153% | 0.0170% | 0.0167% | 0.0156% | 0.0273% |
| Total impurities | ≤2.0% | 0.0579% | 0.0473% | 0.0491% | 0.0670% | 0.0725% | 0.0553 | 0.0760% |

**Table 4: Detection results of related substances in long-term stability test of the composition of Example 4**

| | Limit requirements | Month 0 | Month 3 | Month 6 | Month 9 | Month 12 | Month 18 | Month 24 |
|---|---|---|---|---|---|---|---|---|
| Impurity 1 | ≤1.0% | Not detected | Not detected | Not detected | Not detected | Not detected | Not detected | Not detected |
| Ring-opening impurity | ≤1.0% | 0.0143% | 0.0167% | 0.0163% | 0.0159% | 0.0159% | 0.0240% | 0.0284% |
| Total impurities | ≤2.0% | 0.0520% | 0.0539% | 0.0566% | 0.0639% | 0.0652% | 0.0781% | 0.0716% |

It can be seen from the above results that the compositions provided in Example 1 and Example 4 of the present invention have shown better stability and low impurity content in the accelerated test and long-term stability test. In addition, the present invention adopted the same method to test the compositions provided by Example 2 and Example 3, so the stability was not significantly different from that of Example 1 and Example 4.

### Example 8 Phase I clinical safety research

1. Source of eye drops:
   loxoprofen sodium eye drops: eye drops of different concentrations were prepared according to Example 1; and
   placebo: loxoprofen sodium was not added in Example 1, and the other components were not changed.
2. Research methods: single-center, randomized, double-blind, single and multiple administration dosage escalation designs were used to explore the safety, tolerability and pharmacokinetic characteristics of loxoprofen sodium eye drops. Single administration includes dosages of 0.025 mg (a concentration of 0.5 mg/ml), 0.05 mg (a concentration of 1 mg/ml), 0.1 mg (a concentration of 2 mg/ml), and 0.2 mg (a concentration of 4 mg/mL), with an administration method of once a day, one drop each; and multiple administration includes dosages of 0.05 mg, 0.1 mg, and 0.2 mg, with an administration method of four times a day, one drop each. The pharmacokinetic test data was shown in Table 5.

**Table 5: Pharmacokinetic parameters of loxoprofen in phase I clinical trial**

| **Type of administration** | **Dosage of administration mg** | **Average Cₘₐₓ (ng/mL)** | **Average AUC₀₋ₜ (h*ng/mL)** | **Average Tₘₐₓ (h)** |
|---|---|---|---|---|
| Single administration | 0.1 | 3.69±0.88 | 1.71±0.99 | 0.2 |
| | 0.2 | 8.73±5.19 | 6.82±6.30 | 0.2 |
| Multiple administration | 0.05 | 2.66±0.60 | 0.47±0.43 | 0.2 |
| | 0.1 | 4.22±1.79 | 2.08±1.56 | 0.2 |
| | 0.2 | 9.46±3.73 | 8.33±4.40 | 0.2 |

| | | | | |
|---|---|---|---|---|
| Note: **Cₘₐₓ**: Peak concentration **AUC₀₋ₜ**: Area under the concentration-time curve (0 to t hours) **Tₘₐₓ:** Time to peak concentration | | | | |

Conclusions: The results of the phase I clinical trial showed that there were no systemic adverse reactions after single administration, except for local adverse reactions caused by eye drops. Except for the adverse reactions of eye discomfort after multiple administration, no systemic adverse reactions occurred in either the test drug or placebo. Combined with the pharmacokinetic results in Table 5, it could be seen that the loxoprofen sodium eye drops quickly entered the circulatory system after administration, but the systemic exposure was low; and systemic adverse reactions could quickly return to baseline levels without intervention, indicating that loxoprofen sodium eye drops had good systemic safety.

In addition, according to the results of ocular safety examination of single and multiple administration tests, intraocular pressure and vision were not affected after the administration of the loxoprofen sodium eye drops. There was no significant change from baselines in the results of standard ophthalmological examination and color fundus photography. Ocular tolerance observations showed that the overall incidence of eye discomfort and ocular irritation caused by the eye drops was not high, and could quickly return to normal without intervention. The overall results showed that the loxoprofen sodium eye drops had good ocular safety and were well tolerated at the maximum dosage set in this trial.

Although the present invention has been described in detail with general description, specific embodiments and tests above, some modifications or improvements can be made on the basis of the present invention, which are obvious to those skilled in the art. Therefore, some modifications or improvements made without departing from the spirit of the present invention should fall within the protection scope of the present invention.

## Claims

1. A composition containing loxoprofen sodium, wherein the composition comprises loxoprofen sodium, sodium hyaluronate, and polysorbate 80, wherein the sodium hyaluronate has a molecular weight of 0.8×10⁶-1.6×10⁶.

2. The composition according to claim 1, wherein the sodium hyaluronate has a molecular weight of 0.8×10⁶-1.4×10⁶.

3. The composition according to claim 2, wherein the sodium hyaluronate has a molecular weight of 0.8×10⁶-1.0×10⁶, 1.0×10⁶-1.2×10⁶, or 1.2×10⁶-1.4×10⁶.

4. The composition according to claim 1, wherein the composition has a mass ratio of the loxoprofen sodium to the sodium hyaluronate to the polysorbate 80 of (1-4): (0.5-2): (0.1-1).

5. The composition according to claim 4, wherein the composition has a mass ratio of the loxoprofen sodium to the sodium hyaluronate to the polysorbate 80 of 1: (0.5-1.5): (0.1-1).

6. The composition according to claim 4, wherein the composition has a mass ratio of the loxoprofen sodium to the sodium hyaluronate to the polysorbate 80 of 1: (0.8-1.2): (0.2-0.5).

7. The composition according to any one of claims 1 to 3, wherein the composition further comprises one or more of a metal ion complexing agent, an osmotic pressure regulator, a pH regulator, and a bacteriostatic agent.

8. The composition according to claim 7, wherein
the metal ion complexing agent is selected from disodium edetate; and/or,
the osmotic pressure regulator is selected from one or more of sodium chloride, mannitol, glucose, and potassium chloride; and/or,
the pH regulator is selected from one or more of sodium hydroxide, hydrochloric acid, sodium citrate, citric acid, boric acid, and borax; and/or,
the bacteriostatic agent is selected from one or more of thiomersal, quaternary ammonium salts, Domiphen, chlorhexidine, chlorobutanol, nipagin, and sorbic acid.

9. The composition according to claim 8, wherein the composition comprises loxoprofen sodium, sodium hyaluronate, disodium edetate, sodium chloride, and polysorbate 80.

10. The composition according to claim 9, wherein the composition comprises the following components in parts by weight:
| | |
|---|---|
| loxoprofen sodium | 1-4; |
| sodium hyaluronate | 0.5-2; |
| disodium edetate | 0.05-0.2; |
| sodium chloride | 4-10; and |
| polysorbate 80 | 0.1-1. |

11. The composition according to claim 10, wherein the composition comprises the following components in parts by weight:
| | |
|---|---|
| loxoprofen sodium | 1; |
| sodium hyaluronate | 0.8-1.2; |
| disodium edetate | 0.05-0.1; |
| sodium chloride | 4-9; and |
| polysorbate 80 | 0.2-0.5. |

12. The composition according to claim 9, wherein the composition comprises loxoprofen sodium, sodium hyaluronate, disodium edetate, sodium chloride, polysorbate 80, and benzalkonium chloride.

13. The composition according to claim 12, wherein the composition comprises the following components in parts by weight:
| | |
|---|---|
| loxoprofen sodium | 1-4; |
| sodium hyaluronate | 0.5-2; |
| disodium edetate | 0.05-0.2; |
| sodium chloride | 4-10; |
| polysorbate 80 | 0.1-1; and |
| benzalkonium chloride | 0.05-0.2. |

14. The composition according to claim 13, wherein the composition comprises the following components in parts by weight:
| | |
|---|---|
| loxoprofen sodium | 1; |
| sodium hyaluronate | 0.8-1.2; |
| disodium edetate | 0.05-0.1; |
| sodium chloride | 4-9; |
| polysorbate 80 | 0.2-0.5; and |
| benzalkonium chloride | 0.05-0.1. |

15. The composition according to any one of claims 1 to 14, wherein the composition is in the form of an aqueous solution.

16. The composition according to claim 15, wherein the loxoprofen sodium has a concentration of 0.05-5 mg/mL in the aqueous solution.

17. The composition according to claim 16, wherein the loxoprofen sodium has a concentration of 1-2 mg/mL in the aqueous solution.

18. The composition according to any one of claims 1 to 14, wherein the composition is a solution, a gelling agent, or an ointment.

19. A preparation method for the composition according to any one of claims 1 to 18, wherein, the method comprises the following steps: preparing an aqueous solution of sodium hyaluronate; dissolving other components including loxoprofen sodium in water to obtain a mixture; then mixing the mixture with the aqueous solution of sodium hyaluronate; and adding water to a constant volume.

20. The preparation method according to claim 19, wherein the water used to dissolve the sodium hyaluronate has a temperature of 40°C or less; and/or, a stirring state is maintained in the preparation process.

21. A use of the composition according to any one of claims 1 to 18 in the preparation of a drug for preventing or treating ophthalmic diseases.

22. The use according to claim 21, wherein the ophthalmic disease is an inflammation.

23. The use according to claim 22, wherein the ophthalmic disease is allergic conjunctivitis or uveitis.

24. The use according to claim 21, wherein the ophthalmic disease is a dry eye disease.

25. The use according to claim 24, wherein the ophthalmic disease is a dry eye disease caused by lacrimal gland dysfunction, a dry eye disease caused by reduced tear secretion, a dry eye disease caused by poor tear film stability, or a dry eye disease caused by corneal epithelial cell injury; or
the ophthalmic disease is lacrimal gland dysfunction or is manifested by reduced tear secretion; or
the ophthalmic disease is poor tear film stability; or,
the ophthalmic disease is local edema caused by corneal epithelial cell injury or eye injury.
